(19) Europäisches Patentamt
European Patent Office
Office européen des brevets

(11) **EP 1 932 486 A2**

## (12) EUROPEAN PATENT APPLICATION

(43) Date of publication:
**18.06.2008 Bulletin 2008/25**

(51) Int Cl.:
***A61B 17/32*** *(2006.01)*

(21) Application number: **07122074.3**

(22) Date of filing: **03.12.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **13.12.2006 KR 20060127474**

(71) Applicants:
  • **Curexo Inc.**
    **Gyeonggi-do 431-070 (KR)**
  • **Lee, Eun-Kyue**
    **Seo-gu**
    **Daejeon-city**
    **302-280 (KR)**

(72) Inventors:
  • **Lee, Eun-Kyue,**
    **Suite 802**
    **302-280, Daejeon-city (KR)**

  • **Kim, Jeong-Han,**
    **808-1801 Daewon Apt.**
    **463-717, Gyeonggi-do (KR)**
  • **Song, Chang-Hun,**
    **1301-804 Tanhyunmaul Apt.**
    **411-781, Gyeonggi-do (KR)**
  • **Shin, Sung-Jin**
    **157-015, Seoul-city (KR)**
  • **Park, Young-Bae,**
    **306-1102 Eunhasu Shinsung Apt.**
    **431-734, Gyeonggi-do (KR)**
  • **Park, Jung-Eun,**
    **101-702 Hyundai Sungwoo Apt.**
    **151-029, Seoul-city (KR)**
  • **Kim, Hakk-Shin**
    **422-090, Gyeonggi-do (KR)**

(74) Representative: **Vorberg, Jens et al**
    **Bauer - Vorberg - Kayser,**
    **Patentanwälte,**
    **Lindenallee 43**
    **50968 Köln (DE)**

## (54) Endoscopic tissue dissector

(57) An endoscopic tissue dissector is disclosed. The endoscopic tissue dissector includes a head (2100) for incising and entering tissue, a body (2200) including an endoscope passage part (2230) coupled to the head (2100) and one side coupled to the head, and a handle (2300) coupled to the other side of the body. A transfer direction of a load applied to the handle is substantially identical to a transfer direction of a load applied to the body from the handle.

FIG. 13a

EP 1 932 486 A2

**Description**

**CLAIM FOR PRIORITY**

This application claims priority to Korean Patent Application No. 2006-0127474 filed on December 13, 2006 in the Korean Intellectual Property Office (KIPO), the entire contents of which are hereby incorporated by reference.

**BACKGROUND**

**1. Field of the Invention**

Example embodiments of the present invention relates in general to tissue dissectors, and more specifically to, endoscopic tissue dissectors for surgery using an endoscope.

**2. Description of the Related Art**

A traditional surgery method is performed such that skin and tissue near cancer or a tumor generated in bodily tissue are incised and when cancer or a tumor is shown, problematic tissue is then removed. Such a traditional surgery method has a problem in terms of an aesthetic aspect because an incised skin is large. Also, because even tissue which does not directly relate to a disease is incised and the incised skin is sewed up, it takes a long time for a patient to recover after surgery.

Particularly, in case of breast cancer surgery or breast plastic surgery which is recently being increased, there is a need for minimizing skin to be incised without removing a breast due to women's aesthetic reason in order to minimize a scar after surgery.

That is, in case where after dissecting a breast tissue containing a cancer lesion through a minimum incision, the dissected breast tissue is withdrawn from the human body through the incision and then the incision is sewed up or a shape of a breast is reconstructed using breast prosthesis and then the incision is sewed up, a cancer removing operation which maintains an appearance similar to a normal breast is possible.

In case where the incision is small, since the incision is thin and deep, a surgeon feels difficult to secure a visual field, whereby a surgeon's fatigue is increased and it is difficult to cope with an urgent situation such as bleeding.

There have been attempts for securing a visual field using an endoscope. However, since the incision is easily closed due to inherent elasticity of tissue, it is difficult to insert a tool for widening the small incision, an endoscope and surgery tools through the small incision and to manipulate them through the small incision, and thus such attempts are difficult to realize in terms of a surgery technique.

Devices for endoscopic vessel harvesting other than endoscopic tissue dissectors for removing cancer or a tumor are disclosed in US Patent Nos. 5,667,480, 5,722,934, 5,725,479, 5,902,315, 5,928,135, 5,928,138, 6,036,713, and 6,206,823 and US Patent Publication Nos. 2003/0065348, 2003/0065349, 2004/0106938, and 2004/0186492 (hereinafter, "references").

In the above references, in order to use a vein vessel of a patient's leg, skin near a desired vessel is incised small, devices disclosed in the above references are then inserted through the incision, a location of the vessel is found using an endoscope, and surgery tools are inserted through internal through holes to harvest a desired vessel.

The endoscopic surgery devices disclosed in the above references have only a function for securing a space in desired tissue and guiding an endoscope to the space. However, in such a vessel harvesting surgery, an area subjected thereto is a small vessel viewed by an endoscope, whereas in case of the breast surgery, all or part of breast tissue is continuously dissected, and thus the endoscopic surgery devices disclosed in the above references are difficult to be used for the breast cancer surgery or breast plastic surgery.

For these reasons, in order to apply the endoscopic surgery devices disclosed in the above references to the breast surgery, a discrete tool for dissecting tissue, called a scoop shaped dissector, should be separately inserted to dissect desired tissue.

This method is not currently used in an actual clinical field because of a problem in that since the incision is narrow and a surgeon should simultaneously manipulate an endoscope and a dissector, a surgeon's fatigue is high, the incision may get easily torn, and a precise surgery can not be performed.

In some clinical fields, an operation has been attempted that force is compulsorily applied to the endoscopic surgery devices disclosed in the above references to dissect tissue and remove breast tissue, but the endoscopic surgery devices disclosed in the above references are not originally designed to dissect tissue and thus are inconvenient.

In addition, in case of the endoscopic surgery devices disclosed in the above references, in order to dissect tissue, a lot of force should be applied because a design of an end portion is confronted by high resistance when dissecting tissue, and the endoscopic surgery devices disclosed in the above references may also move in an undesired direction when advancing them, whereby a surgeon's fatigue and a surgery time are increased.

When the breast tissue is dissected along an anatomical tissue plane, the remaining tissues rapidly get recovered, a complication after surgery is small, and an aesthetic shape of breast is excellent. However, the endoscopic surgery devices disclosed in the above references may deviate from the tissue plane due to movement of when moving forward, and the tissue plane may be dissected, roughly torn.

In order to dissect desired tissue, a large load should be applied, but the endoscopic surgery devices disclosed in the above references are difficult to transfer a large load, and when trying to transfer a large load, a load may be transferred to other tissues than desired tissue, thereby damaging or distorting tissue and enlarging the incision.

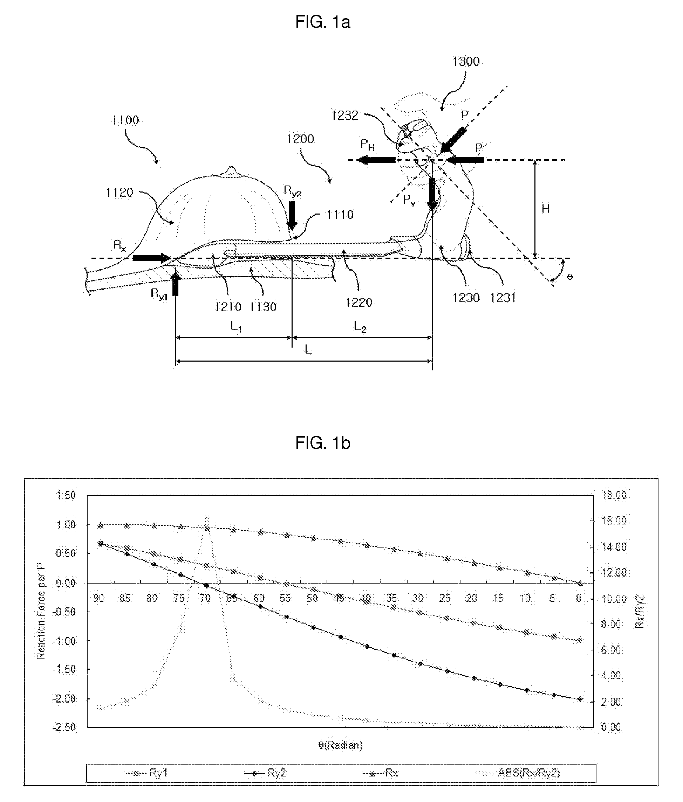

The problems described above are described below with reference to FIGs. 1 a and 1b.

FIG. 1a is a side view illustrating a conventional endoscopic surgery device which is applied to breast cancer surgery. A load applied to tissue is analyzed through FIG. 1a.

A breast 1100 is comprised of an adipose tissue 1120 below skin, and a muscular tissue 1130, a vessel, and other tissues below the adipose tissue which are strongly coupled to each other.

In the breast cancer surgery, a patient lies on an operating table, and a surgeon incises a predetermined side area of a breast near an armpit and inserts an endoscopic surgery device 1200 through the incision.

In a state of grasping a handle 1232 of the endoscopic surgery device 1200 with one hand and a patient's breast with the other hand, a surgeon inserts a head 1210 of the endoscopic surgery device 1200 through an incision of a patient and applies force in an advance direction of the head 1210 by one hand which grasps the handle 1232.

The endoscopic surgery device 1200 includes a body 1220 from which the head 1210 extends and a main body 1230 coupled to the body 1220. The main body 1230 includes an endoscope inserting part 1231 through which an endoscope is inserted up to the head 1210 through the body 1220 and a handle 1232 in which a central axis is formed in a direction for forming a predetermined angle ? with an extending line of the body 1220.

When a surgeon applies a load to the endoscopic surgery device 1200 through the handle 1232, a load P is transferred in a vertical direction to the central axis of the handle 1232, so that reaction forces $R_{y1}$ and $R_{y2}$ which are vertical to an advance direction of the head 1210 are applied to undesired tissue due to a resultant moment.

Below are Equations for the reaction force $R_{y1}$ vertically generated in a tissue-dissected area of a breast and the vertical reaction force $R_{y2}$ working on a patient's incision.

$$(1)\ P_V = P \times \cos? \ ,\ P_H = P \times \sin? \ ,\ R_x = P_H = P \times \sin? \ ,\ L = L_1 + L_2$$

$$(2)\ P_H \times H - P_V \times (L_1 + L_2) - R_{y2} \times L_1 = 0$$

$$(3)\ R_{y2} = P \times (H \times \sin? \ - L \times \cos? \ ) / L_1$$

$$(4)\ R_{y1} = P \times [(H \times \sin? \ - L \times \cos? \ ) / L_1 + \cos? \ ]$$

As can be seen Equation (1) which is a principle of action and reaction, a reaction force $R_x$ applied in an advance direction of the head 1210 is reduced by sin ? due to an angle ? formed by the handle 1232 and the head 1210 or the body 1220.

Also, as can be seen Equations (3) and (4) derived by Equation (2) which is a principle of momentum equilibrium, a principle of momentum equilibrium for a load vector working on the endoscopic surgery device 1200 is applied, so that a value of the reaction force $R_{y1}$ vertical to an advance direction generated in a front end of the head 1210 and a value of the reaction force $R_{y2}$ working on a patient's incised skin exist together.

For example, let us assume that $L_1$ and $L_2$ are 15cm, respectively, ? is 45°, and H is 10cm. The vertical reaction force $R_{y1}$ generated in the front end of the head 1210 reaches about 0.24 times of the load P applied to the handle 1232, and the reaction force $R_{y2}$ working on a patient's incised skin reaches about 0.94 times of the load P applied to the handle 1232.

Also, when a surgeon applies the load P to the handle 1232 in a parallel direction to an advance direction of the head 1210 other than a vertical direction to a central axis of the handle 1232, reaction forces are generated like the flowing equations.

$$(5)\ R_x = P$$

$$(6)\ R_{y1} = 0$$

$$(7)\ R_{y2} = P \times H / L_1$$

If numeric data exemplarily indicated in Equations (1) to (4) are applied, even though the reaction force is reduced compared to when the load P is applied in a vertical direction to a central axis of the handle 1232, the reaction force working on a patient's incised skin still reaches about 0.67 times of the load P applied to the handle 1232.

For these reasons, a surgeon should give his/her thought to a direction of the load P applied to the handle 1232 during surgery and thus feels inconvenient.

FIG. 1b is a graph illustrating the reaction forces $R_x$, $R_{y1}$ and $R_{y2}$ for the working load P when an angle ? formed by the handle 1232 and the head 1210 or the body 1220 is changed from 90° to 0° and a ratio between a parallel reaction force $R_x$ and a reaction force $R_{y2}$ working on incised skin under assumption that a surgeon applies the load P vertically to the handle, based on the above equations and exemplary design data.

In terms of surgery, it is preferable that the parallel reaction force $R_x$ is large and the vertical reaction forces $R_{y1}$ and $R_{y2}$ are small. It is also preferable that the ratio ($R_x/R_{y2}$) between the parallel reaction force $R_x$ and the reaction force $R_{y2}$ working on the incised skin is large.

As shown in FIG. 1b, when an angle ? is around 70°, the largest ratio ($R_x/R_{y2}$) and the smallest reaction force $R_{y2}$ working on the incised skin, which is most sensitive between the vertical reaction forces $R_{y1}$ and $R_{y2}$, are applied.

In order to manufacture the conventional endoscopic surgery device, such a simulation is necessary, but when surgery is performed using the actual endoscopic surgery device, a different result from the simulation result is shown depending on a surgery circumstance and operating method since a simulation condition, e.g., a distance $L_1$ that the endoscopic surgery device is inserted through a patient's incision, may change, whereby there is difficult to optimally design an angle ? formed by the handle 1232 and the head 1210 or the body 1220.

That is, due to the vertical reaction forces $R_{y1}$ and $R_{y2}$ generated by an angle ? formed by the handle 1232 and an advance direction of the head 1210, a large load is applied to a patient's tissue which does not need to be damaged, particularly, to incised skin, thereby increasing an area of the incised skin and unnecessarily damaging undesired tissue.

In addition, in case of the breast cancer surgery or the breast plastic surgery, since lower tissues such as the adipose tissue 1120 and the muscular tissue 1130 are strongly coupled to each other, a large load should be applied to dissect one of the adipose tissue 1120 and the muscular tissue 1130 from the other or an internal part of each of the adipose tissue 1120 and the muscular tissue 1130. Therefore, the endoscopic surgery devices disclosed in the above references can be used only in surgery which does not require a large load such as a vessel harvesting surgery.

Furthermore, since the body 1220 should be formed integrally with the head 1210 which is transparent so that a diseased part can be observed by an endoscope, the body 1220 and the head 1210 are made of transparent plastic. When a large load is applied, the body 1220 may be bent or broken.

## SUMMARY

Accordingly, the present invention is provided to substantially obviate one or more problems due to limitations and disadvantages of the related art.

Example embodiments of the present invention provide an endoscopic tissue dissector in which incised skin is small to minimize a scar after surgery, and damage and death of tissue which is not related to a disease are minimized, thereby reducing a recovery time after surgery.

Example embodiments of the present invention also provide an endoscopic tissue dissector in which a function for guiding an endoscope to a diseased part, securing a visual field of an endoscope and dissecting desired tissue are provided, and a moment generated when a load is transferred to dissect tissue is minimized, thereby minimizing damage and death of undesired tissue caused by a vertical reaction force.

Example embodiments of the present invention also provide an endoscopic tissue dissector in which a body is not bent or broken even by a large load.

Example embodiments of the present invention also provide an endoscopic tissue dissector in which a configuration is simple, thereby reducing the manufacturing cost.

In some example embodiments, an endoscopic tissue dissector includes: a head for incising and entering tissue; a body including an endoscope passage part coupled to the head and one side coupled to the head; and a handle coupled to the other side of the body, wherein a transfer direction of a load applied to the handle is substantially identical to a transfer direction of a load applied to the body from the handle.

In other example embodiments, an endoscopic tissue dissector includes: a head for incising and entering tissue; a body including an endoscope passage part coupled to the head and one side coupled to the head; and a handle coupled to the other side of the body, wherein an angle formed by a central axis direction of a handle and an advance direction of the head is equal to or less than 30 °(degree).

In still other example embodiments, an endoscopic tissue dissector includes: a head for incising and entering tissue; a body including an endoscope passage part coupled to the head and one end coupled to the head; and a handle coupled to the other end of the body, wherein a central axis direction of the handle is substantially identical to a transfer direction of a load applied to the handle.

The handle is formed integrally with the body. The handle includes a first handle part, a second handle part and a coupling part, and the first and second handle parts with a part of the body interposed therebetween are coupled by the coupling part.

The endoscopic tissue dissector further includes a diameter adjusting part having a variable diameter coupled to the endoscope passage part on an opposite side of the handle to the body.

The endoscopic tissue dissector further includes a pipe having higher elasticity than the body which is coupled to the head and inserted into the endoscope passage part.

The head includes a front end part, a middle part, and a rear end part, and the front end part has a pointed shape.

The head includes a front end part, a middle part, and a rear end part, and at least one of the front end part and the middle part has a protruding part which protrudes from a side thereof.

## BRIEF DESCRIPTION OF THE DRAWINGS

Example embodiments of the present invention will become more apparent by describing in detail example embodiments of the present invention with reference to the accompanying drawings, in which:

FIG. 1a is a side view illustrating a conventional endoscopic surgery device which is applied to breast cancer surgery;
FIG. 1b is a load analysis graph obtained by FIG. 1 a
FIGs. 2 and 3 are downward and upward perspective views illustrating a head and a body of an endoscopic tissue dissector according to an exemplary embodiment of the present invention, respectively;
FIGs. 4a and 4b show various exemplary configurations of a handle of the endoscopic tissue dissector according to the exemplary embodiment of the present invention;
FIGs. 5a to 5c are perspective views illustrating various modifications of the head of the endoscope tissue dissector according to the exemplary embodiment of the present invention;
FIG. 6a is a perspective view illustrating an endoscopic tissue dissector according to another exemplary embodiment of the present invention;
FIG. 6b is a cross-sectional view taken along line A-A' of FIG. 6a;
FIGs. 7a to 7c are cross-sectional views illustrating various modifications of a body of the endoscopic tissue dissector according to another exemplary embodiment of the present invention;
FIG. 8 is an exploded perspective view illustrating a reinforcing member for the body according to the exemplary embodiments of the present invention;
FIG. 9 is a perspective view illustrating a handle of the endoscopic tissue dissector according to the exemplary embodiments of the present invention;
FIG. 10a is an enlarged view illustrating an adaptor according to the exemplary embodiments of the present invention;
FIG. 10b is a perspective view illustrating a modification of the adaptor according to the exemplary embodiments of the present invention;
FIG. 11 is a perspective view illustrating the endoscopic tissue dissection of the present invention assembled with an endoscope;
FIG. 12 is a perspective view illustrating a surgery method according to the present invention;
FIG. 13a is a view for analyzing a load applied to tissue according to the present invention; and
FIG. 13b is a load analysis graph obtained by FIG. 13a.

## DESCRIPTION OF EXAMPLE EMBODIMENTS OF THE PRESENT INVENTION

Example embodiments of the present invention are disclosed herein. However, specific structural and functional details disclosed herein are merely representative for purposes of describing example embodiments of the present invention, however, example embodiments of the present invention may be embodied in many alternate forms and should not be construed as limited to example embodiments of the present invention set forth herein.

Accordingly, while the invention is susceptible to various modifications and alternative forms, specific embodiments thereof are shown by way of example in the drawings and will herein be described in detail. It should be understood, however, that there is no intent to limit the invention to the particular forms disclosed, but on the contrary, the invention is to cover all modifications, equivalents, and alternatives falling within the spirit and scope of the invention. Like numbers refer to like elements throughout the description of the figures.

It will be understood that, although the terms first, second, etc. may be used herein to describe various elements, these elements should not be limited by these terms. These terms are only used to distinguish one element from another. For example, a first element could be termed a second element, and, similarly, a second element could be termed a first element, without departing from the scope of the present invention. As used herein, the term "and/or" includes any and all combinations of one or more of the associated listed items.

It will be understood that when an element is referred to as being "connected" or "coupled" to another element, it can be directly connected or coupled to the other element or intervening elements may be present. In contrast, when an element is referred to as being "directly connected" or "directly coupled" to another element, there are no intervening elements present. Other words used to describe the relationship between elements should be interpreted in a like fashion (i.e., "between" versus "directly between", "adjacent" versus "directly adjacent", etc.).

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises", "comprising,", "includes" and/or "including", when used herein, specify the presence of stated features, integers, steps, operations, elements, and/or components, but do not preclude the presence or addition of one or more other features, integers, steps, operations, elements, components, and/or groups thereof.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

It should also be noted that in some alternative implementations, the functions/acts noted in the blocks may occur out of the order noted in the flowcharts. For example, two blocks shown in succession may in fact be executed substantially concurrently or the blocks may sometimes be executed in the reverse order, depending upon the functionality/acts involved.

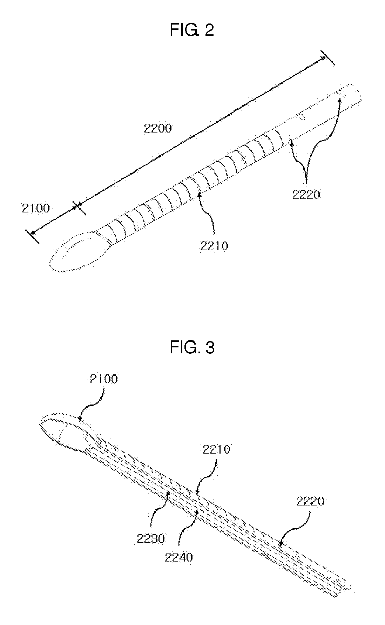

FIGs. 2 and 3 are downward and upward perspective views illustrating a head 2100 and a body 2200 of an endoscopic tissue dissector according to an exemplary embodiment of the present invention, respectively.

The head 2100 is injection-molded of a plastic material which is harmless to the human body and has an elliptical or hemi-spherical structure. The head 2100 includes a front end part which has a predetermined shape for dissecting and wedging and entering incised tissue and a middle part which has the wider width than the front end part to widely open tissue.

The head 2100 has a circular curved upper surface. Preferably, for the breast surgery, the length of the head 2100 is about 5cm, and the width of the middle part is about 3cm. The dimensions of the length of the head 2100 and the width of the middle part can vary depending on a kind of surgery in which the endoscopic tissue dissector of the present invention is used.

However, when the width of the middle part is too large, the incised skin through which the endoscopic tissue dissector of the present invention is inserted is large, and so there is a disadvantage in aesthetic aspect, whereas when the width of the middle part is too small, other surgery tools are difficult to be inserted through the incised skin, and so an effect of the endoscopic tissue dissector for dissecting tissue is reduced.

When the length of the head 2100 is too large, there is a possibility to make a hole in tissue, whereas when the length of the head 2100 is too small, a lot of force is required to dissect tissue. Therefore, the head 210 preferably has a streamline shape.

Preferably, the head 2100 is made of a transparent material so that a surgeon can observe a diseased part through a camera of an endoscope connected up to the head 2100 through an endoscope passage part 2230 of the body 2200. The head 2100 may be made of a colored material if necessary.

Preferably, the body 2200 has the length of about 25 cm and the width of about 2 cm, but the dimensions may be variously designed since a patient's physical structure and size are various.

The body 2200 is coupled to a rear end of the head 2100. Preferably, the body 2200 is formed integrally with the head 2100 such that the endoscope passage part 2230 and/or a guide rail 2240 are coupled to the head 2100. However, the body 2200 may be mounted separately from the head 2100.

Preferably, the body 2200 is made of transparent or colo red plastic which is harmless to the human body like the head 2100.

The endoscope passage part 2230 for coupling front and rear end parts of the body 2200 serves to guide a camera mounted on a front end of an endoscope tube inserted through a handle coupled to the rear end part of the body 2200 up to the head 2100, and the guide rail 2240 serves to guide a surgery tool such as incision scissors or hemostats used during surgery to a diseased part.

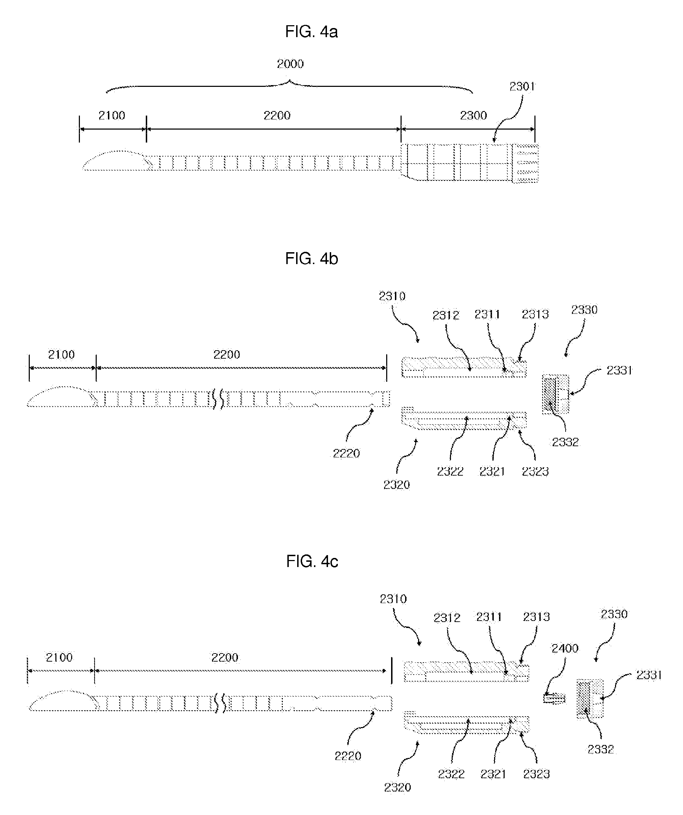

A lock groove 2220 formed in the rear end part of the body 2200 is used to increase clamping force between the handle and the body 2200 as shown in FIGs. 4a to 4c. Preferably, a plurality of concave grooves are formed on bottom and/or top surfaces of the body 2200 as the lock groove 2220.

When the body 2200 is formed integrally with the handle as shown in FIG. 4a, the lock groove 2220 may not be formed.

A scale 2210 is formed on a surface of the body 2200 so that a surgeon can recognize how deep the endoscopic tissue dissector is inserted through the incision when inserting and advancing the endoscopic tissue dissector.

The scale 2210 may be printed or formed in a concave-convex form. Preferably, since the scale 2210 contacts tissue, the scale 2210 of the concave-convex form is preferable.

FIGs. 4a and 4b show various exemplary configurations of the handle of the endoscopic tissue dissector according to the exemplary embodiment of the present invention. The handle may be configured integrally with the body 2200 or may be configured separately from the body 2200 without having an endoscope diameter adjusting means. Alternatively, the handle may have a structure of being separated from the body 2200 and having an endoscope diameter adjusting means.

FIG. 4a shows an endoscopic tissue dissector in which the body 2200 is formed integrally with the handle 2300. A slide preventing part 2301 is formed on a surface of the handle 2300 to prevent a surgeon's hand grasping the endoscopic tissue dissect from being slid, and a through hole (not shown) through which an endoscope is inserted from a rear end of the handle 2300 is formed inside the handle 2300. The through hole is formed to be communicated with the endoscope passage part 2230 of the body 2200.

The slide preventing part 2301 may have a concave-convex or uneven structure and may be formed by performing a surface process for increasing friction force or by attaching a tape having high friction force thereonto.

FIG. 4b shows an endoscopic tissue dissector in which the body 2200 is separated from the handle 2300. The handle 2300 includes first and second handle parts 2310 and 2320 and may further include a coupling part 2330 for coupling the first and second handle parts 2310 and 2320.

Either or both of the first and second handle parts 2310 and 2320 include a slide preventing part 2301 for preventing a surgeon's hand grasping the handle from being slid, a concave groove through which an endoscope inserted from the rear end of the handle 2300 is guided to the endoscope passage part 2230 of the body 2200, a locking protrusion for preventing the handle 2300 from being slid corresponding to the locking groove 2220 of the body 2200, and a male screw groove corresponding to a female screw groove 2332 of the coupling part 2330 coupled to the rear end of the handle to couple the first and second handle parts 2310 and 2320.

Here, concave grooves 2312 and 2322, locking protrusions 2311 and 2321, and male screw grooves 2313 and 2322 may be formed in either or both of the first and second handle parts 2310 and 2320. Preferably, the first handle part 2310 includes a first concave groove 2312, a first locking protrusion 2311, and a first male screw groove 2313, and the second handle part 2320 includes a second concave groove 2322, a second locking protrusion 2321, and a second male screw groove 2323.

The coupling part 2330 includes a through hole 2331 formed on its rear end through which an endoscope tube is inserted. The through hole 2331 extends to a front end of the coupling part 2330 and guides the inserted endoscope tube to the endoscope passage part 2230 of the body 2200 through a space formed by the first and second concave grooves 2312 and 2322.

FIG. 4c shows an endoscopic tissue dissector with an adaptor according to the exemplary embodiment of the present invention. The adaptor of FIG. 4c adjusts its diameter corresponding to the through hole 2331 through which the endoscope tube is inserted so that the endoscope tubes of various diameters can be used.

An adaptor 2400 has a penetrated inside and a slit that its outside is partially cut. The adaptor 2400 is formed in a shape that a cross-sectional external diameter is gradually smaller in a direction for facing the coupling part 2330.

The adaptor 2400 is located between the first and second handle parts 2310 and 2320 and the coupling part 2330. When the coupling part 2330 moves forward the first and second handle parts 2310 and 2320 such that the male screw groove 2313 of the handle 2300 and the female screw groove 2332 of the coupling part 2330 are fastened together by turning the coupling part 2330, a cross-sectional external diameter of the adaptor 2400 is gradually larger, and so a cross-sectional inside diameter of the through hole 2331 is smaller by the slit of the adaptor 2400.

At this time, the through hole 2331 of the coupling part 2330 is configured such that its inside diameter is gradually larger in a direction of from a portion of through which the endoscope tube is inserted to a portion facing the adaptor 2400.

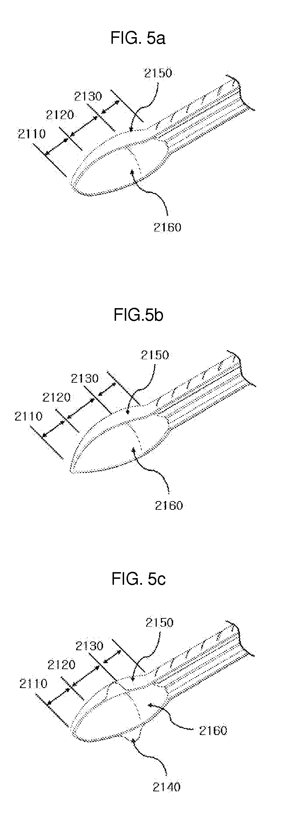

FIGs. 5a to 5c are perspective views illustrating various modifications of the head of the endoscope tissue dissector according to the exemplary embodiment of the present invention. The head 2100 includes a front end part 2110 for incising or dissecting tissue to advance the endoscopic tissue dissector during surgery, a middle part 2120 for more widening the tissue incised or dissected by the front end part 2110, and a rear end part 2130 coupled to the body 2200.

The apex of the front end part 2110 has a streamline shape as shown in FIG. 5a or a pointed shape as shown in FIG. 5b, especially when looking down from the top.

The streamline shaped front end part 2110 does not unnecessarily damage other tissues than desired tissue which are located in a path through which the endoscope tissue dissector of the present invention passes.

The pointed front end part 2110 is used to have the endoscope tissue dissector to easily arrive at a diseased part when a large load is required to dissect strong tissue.

Also, as shown in FIG. 5c, protruding parts 2140 of a wing form may be formed in the middle part 2120 and/or the front end part 2110 in order to more efficiently widen an tissue area to be widened by the middle part 2120 as shown in FIG. 5c. Preferably, the protruding parts 2140 are formed on both sides of a hemi-spherical lower end of the head 2100 as shown in FIG. 5c.

The head 2100 also includes an upper cover 2150 for forming a shape of the head 2100 and an endoscope resting part 2160 in which its inside is depressed and a camera of an endoscope inserted through the endoscope passage part 2230 of the body 2200 is rested.

The endoscope resting part 2160 is communicated with both of the endoscope passage part 2230 and the guide rail 2240 of the body 2200.

A surgeon can observe a diseased part below the head 2100 or a diseased part above the head 2100 through the upper cover 2150 made of a transparent material by using a camera of an endoscope inserted to the endoscope resting part 2160 through the endoscope passage part of the body 2200 during surgery.

Also, a surgeon places various surgery tools such as surgery scissors or hemostats inserted to the endoscope resting part 2160 by the guide rail 2240 of the body 2200 onto a diseased part to perform an operation while observing a diseased part.

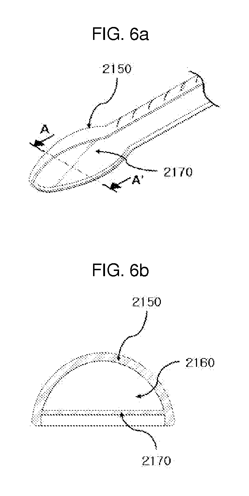

FIG. 6a is a perspective view illustrating an endoscopic tissue dissector according to another exemplary embodiment of the present invention, and FIG. 6b is a cross-sectional view taken along line AA' of FIG. 6a. The endoscopic tissue dissector according to another exemplary embodiment of the present invention further includes a lower cover 2170 formed on the endoscope resting part 2160 of the head 2100 in addition to a configuration of the endoscopic tissue dissector of FIGs. 5a to 5c.

The endoscope resting part 2160 has a closed structure isolated from the outside by the upper cover 2150 and the lower cover 2170, and thus prevented is a phenomenon that a camera lens of an endoscope rested on the endoscope resting part 2160 is smeared with substances such as blood generated during surgery.

Here, a portion of the lower cover 217 communicated with the guide rail 2240 of the body 2200 is not closed, so that a surgery tool inserted through the guide rail 2240 is transferred to a diseased part during surgery.

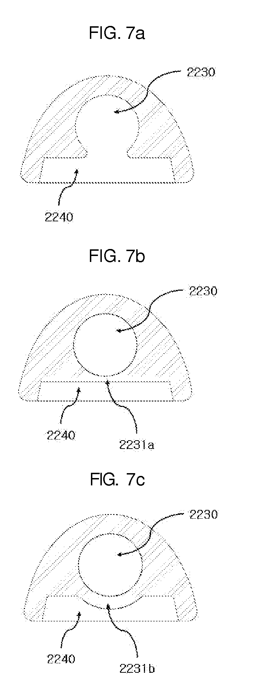

The lower cover 2170 is also formed on the body 2200 to extend to an isolation layer for isolating the endoscope passage part 2230 from the guide rail 2240 as shown in FIGs. 7b and 7c which will be described later in detail.

The lower cover 2170 is preferably made of a transparent material so that an endoscope can take a photograph of a location of a surgery tool and a diseased part, and the lower cover 2170 is more preferably made of the same material as the upper cover 2150.

FIGs. 7a to 7c are cross-sectional views illustrating various modifications of the body of the endoscopic tissue dissector according to another exemplary embodiment of the present invention. The body 2200 includes the endoscope passage part 2230 through which an endoscope is transferred and the guide rail 2240 through which surgery tools are transferred.

In FIG. 7a, the endoscope passage part 2230 is communicated with the guide rail 2240. Such a body configuration is profitable when the height of the body 220 is low but the endoscope passage part 2230 of the larger diameter is required since the diameter of an endoscope to be inserted is large or when there are a lot of surgery tools to be transferred or the guide rail 2240 of the high height is required since the diameter or thickness of a surgery tool is large.

In FIGs. 7b and 7c, the body 2200 further includes an isolation layer 2231 a or 2231 b for isolating the endoscope passage part 2230 from the guide rail 2240. An isolation layer configuration of FIG. 7b is profitable when a lot of surgery tools are transferred or a large surgery tool is transferred since a space of the guide rail 2240 is relatively large, but the isolation layer 2231 a may be broken when the body 2200 is bent by a large load applied to reach the endoscopic tissue dissector to a diseased part since the isolation layer 2231 a is weak.

Since the isolation layer 2231 b of FIG. 7c is thick in thickness and a space of the guide rail 2240 is relatively small, it is difficult to transfer a lot of surgery tools or a large surgery tool, compared to that of FIG. 7b. However, even though the body 2200 is bent, the isolation layer 2231 b is not easily broken and thereby prevents the body 2200 from being bent, serving as a reinforcing member of reinforcing the body 2200.

The isolation layer 2231 a or 2231 b may be formed integrally with the body 2200 or separately from the body 2200. In latter case, the isolation layer may be coupled to the body 2200 by using a medical adhesive which is harmless to the human body.

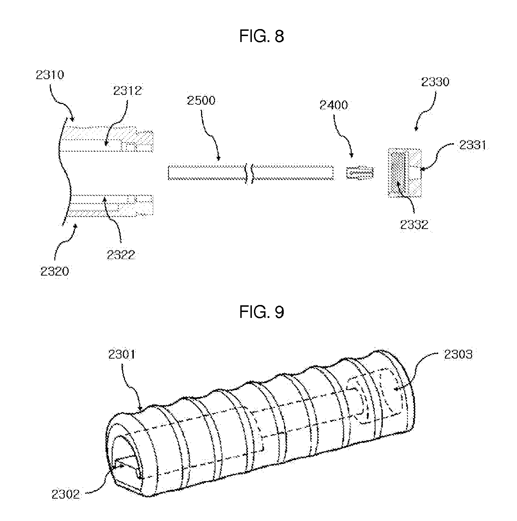

FIG. 8 is an exploded perspective view illustrating a reinforcing member for the body according to the exemplary embodiments of the present invention. As shown in FIGs. 7a to 7c, the endoscopic tissue dissector may further include an additional reinforcing member for reinforcing the body 2200 since the body 220 made of plastic may be easily bent or broken when a large load is applied to reach the endoscopic tissue dissector to a diseased part.

The reinforcing member 2500 has an external diameter smaller than an inside diameter of the endoscope passage part 2230 to be inserted into the endoscope passage part 2230 of the body 2200 and is formed in a pipe form with an inside diameter enough for an endoscope to be transferred through the reinforcing member 2500.

The length of the reinforcing member 2500 preferably corresponds to the length of the body 2200 and more preferably corresponds to a distance that extends from a point that the body 2200 is coupled to the lower end 2130 of the head 2100 to the through hole 2331 of the handle 2300 through which an endoscope is inserted in a state that the head 2100, the body 2200 and the handle 2300 are coupled.

In order to prevent the reinforcing member 2500 from being inserted up to the endoscope resting part 2160 of the head 2100, a protruding part (not shown) may be further formed in a portion of the endoscope passage part 2230 corresponding to a location that the head 2100 and the body 2200 are coupled to each other.

Preferably, the reinforcing member 2500 is made of corrosion-resistant metal having a higher elastic coefficient than the body 2200.

For example, the reinforcing member 2500 is made of stainless steel, a nickel-chromium alloy or high strength plastic.

FIG. 9 is a perspective view illustrating the handle of the endoscopic tissue dissector according to the exemplary embodiments of the present invention.

The slide preventing part 2310 is formed on a surface of the handle to prevent a surgeon's hand grasping the handle from being slid. A pass hole through which an endoscope passes is formed inside the handle. A body coupling part 2302 coupled to the body 2200 is formed in the front end part of the handle in a shape corresponding to a cross section of the body 2200 shown in FIGs. 7a to 8b, contacting the guide rail 2240.

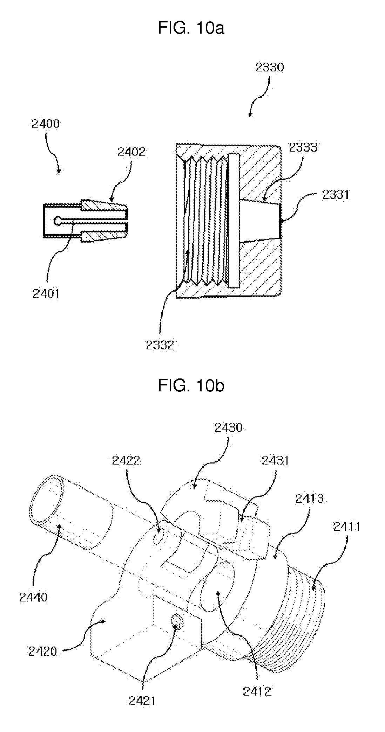

A female screw hole 2303 is formed in the rear end part of the handle to be coupled to the adaptor 2400 of FIG. 10b.

The female screw hole 2303 and the pass hole are communicated with each other inside the handle 2300.

FIG. 10a is an enlarged view illustrating the adaptor according to the exemplary embodiments of the present invention.

An adaptor 2400 includes a diameter adjusting part 2401 having a slit for adjusting a cross-sectional diameter and an inclined part 2402 whose external diameter is gradually smaller corresponding to the coupling part 2330.

A through hole is formed inside the adaptor 2400 so that an endoscope tube inserted from the coupling part 2330 can pass through.

The through hole 2331 of the coupling part 2330 formed corresponding to the inclined part 2402 of the adaptor 2400 includes an inside inclined surface 2333 whose inside diameter is gradually larger in a direction facing the adaptor 2400.

When the endoscopic tissue dissector of the present invention is assembled, the inside inclined surface 2333 of the coupling part 2330 presses the inclined part 2402 of the adaptor 2400 by the female screw groove 2332 of the coupling part 2330 and thus as a gap of the slit as the diameter adjusting part 2401 is narrower, the inside diameter of the adaptor 2400 is smaller, and to the contrary the inside diameter of the adaptor 2400 is larger by turning the coupling part 2330, whereby endoscope tubes with various diameters can be used.

FIG. 10b is a perspective view illustrating a modification of the adaptor according to the exemplary embodiments of the present invention.

A male screw 2411 is coupled to the female screw hole 2303 of the handle 2300 of FIG. 9, and an endoscope through hole 2412 is formed inside a flange 2413.

One end of a hinge shaft 2422 is coupled to the flange 2413, and first and second clamps 2420 and 2430 are pivotally coupled to the other end of the hinge shaft 2422. A clamp screw hole 2421 is formed in the first clamp 2420, and a coupling groove 2431 is formed in the second clamp 2430. Therefore, the coupling groove 2431 and the clamp screw hole 2412 are fastened or loosed by a discrete screw (not shown).

An elastic slave 2440 is positioned in an inside diameter surface of the first and second clamps 2420 and 2430. The elastic slave 2440 serves to prevent relative slide and relative distortion with an endoscope (or endoscope tube) and is preferably made of a rubber material.

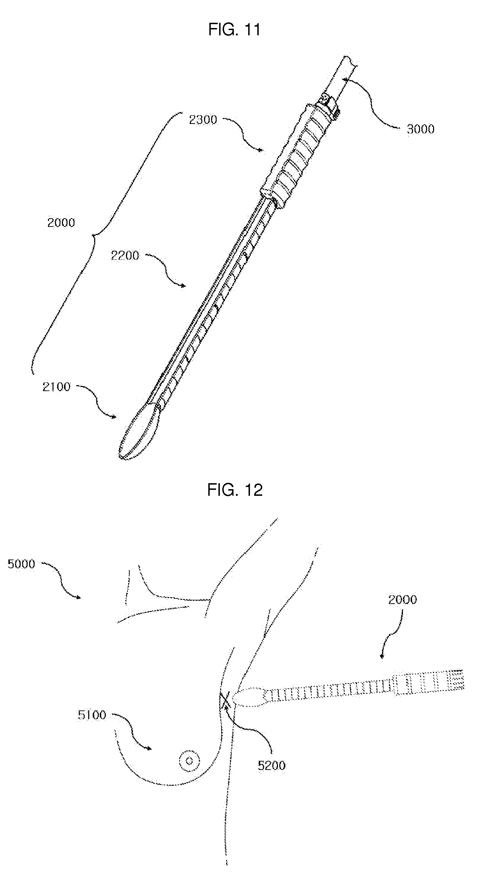

FIG. 11 is a perspective view illustrating the endoscopic tissue dissection of the present invention assembled with an endoscope.

An endoscope tube 3000 is coupled to an adaptor, and the head 2100 and a part of the body 220 enter the human body. At this time, an endoscope extends from the endoscope tube 300 to the head 2100, and an endoscope camera located on a front end of an endoscope is rested in the endoscope resting part 2160 of the head 2100.

FIG. 12 is a perspective view illustrating a surgery method according to the present invention. FIG. 12 exemplarily shows breast surgery such as breast cancer surgery or breast plastic surgery.

Before surgery, a surgeons diagnoses a diseased part which the endoscopic tissue dissector 2000 of the present invention is to reach, determines a location of an incision 5200 around a patient's armpit through which the endoscopic tissue dissector is to be inserted, and incises corresponding skin by using a surgical knife to create the incision 5200.

Thereafter, a surgeon grasps the handle 2300 of the endoscopic tissue dissector 200 with one hand, inserts the head of 2100 of the endoscopic tissue dissector 200 through the incision 5200 and applies a load toward a diseased part in an advance direction.

At this time, a surgeon grasps a breast 5100 with the other hand which does not grasp the endoscopic tissue dissector to feel movement of the endoscopic tissue dissector 2000 and determines whether the endoscopic tissue dissector 2000 reaches a diseased part or not through an endoscope while advancing the endoscopic tissue dissector 2000.

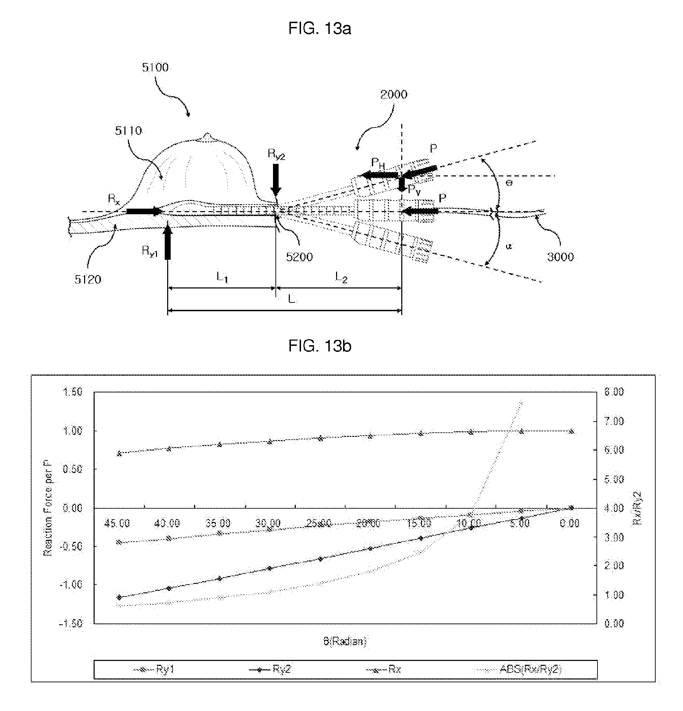

FIG. 13a is a view for analyzing a load applied to tissue according to the present invention.

The breast 5100 includes a first tissue 5110 which has an adipose tissue below skin and a second tissue 5120 having other tissues such as a muscular tissue and vessel below the first tissue 5110 which are strongly coupled to each other.

In the breast surgery such as the breast cancer surgery or the breast plastic surgery, a patient 5000 lies on an operating table, and a surgeon incises a predetermined side area of a breast near an armpit by using a surgical knife to creat the incision 5200, inserts the endoscopic surgery device 2000 through the incision 5200, and applies a load P in an advance direction of the endoscopic tissue dissector 2000, whereby the endoscopic tissue dissector 2000 advances while dissecting desired tissue through the head 2100.

At this time, action force and reaction force generated when the load P is applied to advance the endoscopic tissue dissector 2000 are simplified to compare a load transferred to tissue.

When the body 2200 of the endoscopic tissue dissector 2000 is bent or transformed, an angle ? formed between a central axis direction of the handle 2300 and an advance direction of the head 2100 has been simulated.

Below are Equations about parallel reaction force Rx and vertical reaction force Ry1 which work on the front end part 2110 of the head 2100 and vertical reaction force Ry2 working on a patient's incision 5200 when the endoscopic tissue dissector 2000 advances while dissecting desired tissue.

$$\text{(a) } P_V = P \times \sin? \ , \ P_H = P \times \cos? \ , \ R_x = P_H = P \times \cos? \ , \ L = L_1 + L_2$$

$$\text{(b) } P_H \times H - P_V \times L - R_{y2} \times L_1 = 0$$

$$\text{(c) } R_{y2} = P \times (H \times \cos? \ - L \times \sin? \ ) / L_1$$

$$\text{(d) } R_{y1} = R_{y2} + P_V = P \times [(H \times \cos? \ - L \times \sin? \ ) / L_1 + \sin? \ ]$$

When the angle ? formed between a central axis direction of the handle 2300 and an advance direction of the head 2100 is zero (0), that is, the body 2200 of the endoscopic tissue dissector 200 is not bent, the vertical reaction force $R_{y1}$ working on the front end part 2110 of the head 2100 and the vertical reaction force $R_{y2}$ working on a patient's incision 5200 are zero (0), and the parallel reaction force $R_x$ working on the front end part 2110 of the head 2100 is equal to the load P which a surgeon applies to the handle 2300.

In the above Equations, "H" denotes a height difference between an advance direction axis of the head 2100 and a load transferring center of the handle 2300 when it is assumed that a center of the handle 2300 is a load transferring center and the body 220 is bent at the incision 5200 in a straight line form.

The vertical reaction force $R_{y2}$ working on the incision 5200 is derived by Equation (c) based on a principle of momentum equilibrium of Equation (b), and the vertical reaction force $R_{y1}$ working on the front end part 2110 of the handle 2100 is derived by Equation (d).

FIG. 13b is a load analysis graph obtained by FIG. 13a.

In order to compare with the conventional endoscopic tissue dissector, it is assumed that in the endoscopic tissue dissector of the present invention, the length L is 30 cm, and the length $L_1$ that the endoscopic tissue dissector is inserted into tissue is 15 cm.

The graph of FIG. 13 shows reaction forces $R_x$, $R_{y1}$, and $R_{y2}$ for a working load P when an angle ? formed by the load transferring axis direction of the handle 2300 and the advance direction of the head 2100 is changed from 45° to 0° and ratio ABS($R_x/R_{y2}$) between the parallel reaction force $R_x$ and the reaction force $R_{y2}$ working on incised skin under assumption that a surgeon applies a load P to the handle 2300, based on the above Equations and exemplary design data of FIG. 13a.

The angle ? of 0° is most preferable since it is more preferable as the vertical reaction forces $R_{y1}$ and $R_{y2}$ are closer to zero (0), as the parallel reaction force $R_x$ is closer to one (1), and as the ratio ABS($R_x/R_{y2}$) between the parallel reaction force $R_x$ and the reaction force $R_{y2}$ working on incised skin is closer to infinity.

In order for the angle ? formed by the load transferring axis direction of the handle 2300 and the advance direction of the head 2100 to be zero (0), the body 220 should not be bent. To this end, the reinforcing member 2500 of FIG. 8 or the isolation layer 2231 b of FIG. 7c are used for the body 2200 not to be bent by the working load P when the endoscopic tissue dissector 2000 is advanced to dissect desired tissue.

Since it is preferable that the ratio ABS($R_x/R_{y2}$) between the parallel reaction force $R_x$ and the reaction force $R_{y2}$ working on incised skin is one (1) or more, the angle ? formed by the load transferring axis direction of the handle 2300 and the advance direction of the head 2100 is preferably equal to or less than 30°.

In the load P, the reaction forces $R_x$, $R_{y1}$, and $R_{y2}$ and the ratio ABS($R_x/R_{y2}$), a negative value means that a working direction is an opposite direction of a working direction of the load shown in FIG. 13a.

The reaction force which is most importantly considered among the reaction forces $R_x$, $R_{y1}$, and $R_{y2}$ is the vertical reaction force $R_{y2}$. It is because the incision 5200 needs to be minimized due to an aesthetic reason, but when the vertical reaction force $R_{y2}$ working on the incision 5200 is large, the area of the incision is expanded. In particularly, skin tissue is easily torn since a load is concentrated on a portion incised by a surgical knife.

Therefore, the angle ? is preferably designed in terms of a ratio between the reaction force $R_{y2}$ working on the incision 5200 and the parallel reaction force $R_x$ which is a driving force for advancing the endoscopic tissue dissector to dissect desired tissue.

In this specification, a term "dissect" means to widen a portion between tissues of the same kind or a portion between tissues of different tissues, and a term "dissector" means a tool for wedging into and entering a portion between tissues of the same kind or a portion between tissues of different tissues to be widened.

As described above, the endoscopic tissue dissector of the present invention has the following advantages.

Firstly, incised skin is small to minimize a scar after surgery, and damage and death of tissue which is not related to a disease are minimized, thereby reducing a recovery time after surgery.

Secondly, a moment generated when a load is transferred to dissect tissue is minimized, thereby minimizing damage and death of undesired tissue caused by a vertical reaction force.

Thirdly, the body is not bent or broken even by a large load.

Lastly, a configuration is simple, thereby reducing the manufacturing cost.

While the example embodiments of the present invention and their advantages have been described in detail, it should be understood that various changes, substitutions and alterations may be made herein without departing from the scope of the invention.

**Claims**

1.  An endoscopic tissue dissector, comprising:

    a head for incising and entering tissue;
    a body including an endoscope passage part coupled to the head and one side coupled to the head; and
    a handle coupled to the other side of the body,

    wherein a transfer direction of a load applied to the handle is substantially identical to a transfer direction of a load applied to the body from the handle.

**2.** The endoscopic tissue dissector of claim 1, wherein the handle is formed integrally with the body.

**3.** The endoscopic tissue dissector of claim 1, wherein the handle comprises a first handle part, a second handle part and a coupling part, and the first and second handle parts with a part of the body interposed therebetween are coupled by the coupling part.

**4.** The endoscopic tissue dissector of claim 1, further comprising, a diameter adjusting part having a variable diameter coupled to the endoscope passage part on an opposite side of the handle to the body.

**5.** The endoscopic tissue dissector of claim 1, further comprising, a pipe having higher elasticity than the body which is coupled to the head and inserted into the endoscope passage part.

**6.** The endoscopic tissue dissector of claim 1, wherein the head comprises a front end part, a middle part, and a rear end part, and the front end part has a pointed shape.

**7.** The endoscopic tissue dissector of claim 1, wherein the head comprises a front end part, a middle part, and a rear end part, and at least one of the front end part and the middle part has a protruding part which protrudes from a side thereof.

**8.** An endoscopic tissue dissector, comprising:

> a head for incising and entering tissue;
> a body including an endoscope passage part coupled to the head and one side coupled to the head; and
> a handle coupled to the other side of the body,

wherein an angle formed by a central axis direction of a handle and an advance direction of the head is equal to or less than 30 ° (degree).

**9.** The endoscopic tissue dissector of claim 8, wherein the handle is formed integrally with the body.

**10.** The endoscopic tissue dissector of claim 8, wherein the handle comprises a first handle part, a second handle part and a coupling part, and the first and second handle parts with a part of the body interposed therebetween are coupled by the coupling part.

**11.** The endoscopic tissue dissector of claim 8, further comprising, a diameter adjusting part having a variable diameter coupled to the endoscope passage part on an opposite side of the hand le to the body.

**12.** The endoscopic tissue dissector of claim 8, further comprising, a pipe having higher elasticity than the body which is coupled to the head and inserted into the endoscope passage part.

**13.** The endoscopic tissue dissector of claim 8, wherein the head comprises a front end part, a middle part, and a rear end part, and the front end part has a pointed shape.

**14.** The endoscopic tissue dissector of claim 8, wherein the head comprises a front end part, a middle part, and a rear end part, and at least one of the front end part and the middle part has a protruding part which protrudes from a side thereof.

**15.** An endoscopic tissue dissector, comprising:

> a head for incising and entering tissue;
> a body including an endoscope passage part coupled to the head and one end coupled to the head; and
> a handle coupled to the other end of the body,

wherein a central axis direction of the handle is substantially identical to a transfer direction of a load applied to the handle.

**16.** The endoscopic tissue dissector of claim 15, wherein the handle is formed integrally with the body.

**17.** The endoscopic tissue dissector of claim 15, wherein the handle comprises a first handle part, a second handle part and a coupling part, and the first and second handle parts with a part of the body interposed therebetween are coupled by the coupling part.

**18.** The endoscopic tissue dissector of claim 15, further comprising, a diameter adjusting part having a variable diameter coupled to the endoscope passage part on an opposite side of the handle to the body.

**19.** The endoscopic tissue dissector of claim 15, further comprising, a pipe having higher elasticity than the body which is coupled to the head and inserted into the endoscope passage part.

**20.** The endoscopic tissue dissector of claim 15, wherein the head comprises a front end part, a middle part, and a rear end part, and the front end part has a pointed shape.

**21.** The endoscopic tissue dissector of claim 15, wherein the head comprises a front end part, a middle part, and a rear end part, and at least one of the front end part and the middle part has a protruding part which protrudes from a side thereof.

## FIG. 1a

## FIG. 1b

FIG. 2

2200

2100

2220

2210

FIG. 3

2100

2210

2220

2230

2240

FIG. 4a

FIG. 4b

FIG. 4c

FIG. 5a

FIG.5b

FIG. 5c

FIG. 6a

FIG. 6b

FIG. 7a

2230

2240

FIG. 7b

2230

2240

2231a

FIG. 7c

2230

2240

2231b

FIG. 8

FIG. 9

FIG. 10a

FIG. 10b

FIG. 11

FIG. 12

FIG. 13a

FIG. 13b

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20060127474
- US 5667480 A
- US 5722934 A
- US 5725479 A
- US 5902315 A
- US 5928135 A
- US 5928138 A
- US 6036713 A
- US 6206823 B
- US 20030065348 A
- US 20030065349 A
- US 20040106938 A
- US 20040186492 A